Europäisches Patentamt

(19) European Patent Office

Office européen des brevets

(11) Numéro de publication : **0 304 354 B1**

(12) **FASCICULE DE BREVET EUROPEEN**

(45) Date de publication du fascicule du brevet :
23.10.91 Bulletin 91/43

(51) Int. Cl.$^5$ : **C11B 3/00, A61K 31/23**

(21) Numéro de dépôt : **88401868.0**

(22) Date de dépôt : **20.07.88**

(54) **Procédé de purification de l'huile d'olive.**

Le dossier contient des informations
techniques présentées postérieurement au
dépôt de la demande et ne figurant pas dans le
présent fascicule.

(30) Priorité : **23.07.87 FR 8710404**

(43) Date de publication de la demande :
**22.02.89 Bulletin 89/08**

(45) Mention de la délivrance du brevet :
**23.10.91 Bulletin 91/43**

(84) Etats contractants désignés :
**AT BE CH DE FR GB IT LI LU NL SE**

(56) Documents cités :
**FR-A- 891 271**
**GB-A- 642 751**

(73) Titulaire : **NB INTERNATIONAL
TECHNOLOGIES
Avenue Nestlé 55
CH-1800 Vevey (CH)**

(72) Inventeur : **Melin, Christian
1, Square des Ecrivains
F-91370 Verrières le Buisson (FR)**

(74) Mandataire : **Thouret-Lemaitre, Elisabeth et al
SYNTHELABO Service Brevets 22, avenue
Galilée
F-92352 LE PLESSIS ROBINSON CEDEX (FR)**

EP 0 304 354 B1

## Description

La présente invention a pour objet un procédé de purification de l'huile d'olive.

Le procédé de l'invention permet d'obtenir à partir d'une huile d'olive vierge extra de première pression une huile d'olive purifiée utilisable en nutrition entérale ou parentérale.

L'huile obtenue est une huile d'olive de très faible acidité libre, non oxydée et exempte des pigments responsables de la coloration.

L'huile obtenue a des qualités nutritionnelles supérieures à l'huile d'olive vierge telle que décrite dans la Pharmacopée Européenne 2e édition partie II.10.

La purification d'huiles a déjà été décrite dans la littérature, par exemple dans le brevet français 891271 et dans le brevet britannique 642751.

L'huile obtenue selon le procédé de l'invention peut être utilisée dans des émulsions que l'on peut administrer par voie entérale ou parentérale, par exemple dans la fabrication des émulsions décrites par la Demanderesse dans ses brevets français 87.10407 et 87.10405.

Le procédé de l'invention consiste à purifier une huile d'olive répondant à la définition de la Pharmacopée Européenne en trois étapes :

— Neutralisation par une solution aqueuse saturée de carbonate alcalin (disodique ou dipotassique) équivalent à deux à 10 fois le poids d'acide oléique calculé après dosage, préférentiellement 2 à 3 fois.

Cette opération est réalisée sous atmosphère inerte à une température comprise en 20 et 80°C, préférentiellement 40 à 50°C, sous agitation.

Après décantation et séparation de la phase aqueuse, l'huile est lavée à l'eau distillée jusqu'à neutralité complète.

— Décoloration : après séchage, on ajoute à l'huile neutralisée, maintenue sous azote à une température comprise entre 20 et 80°C (préférentiellement 50 à 60°C), de 1 à 10% en poids de terre décolorante (préférentiellement 5 à 8%), pendant 30 à 40 minutes, sous agitation lente (100 à 500 r.p.min).

— Désodorisation dans le cas où cette opération s'avère nécessaire. Cette opération est réalisée à basse température : $\leq 190°C$ sous un vide poussé ($1.333 \times 10^{-1}$ Pa ($10^{-3}$ Torr) environ).

L'exemple suivant illustre l'invention : on neutralise l'huile d'olive répondant à la définition de la Pharmacopée Européenne à 45°C par une solution aqueuse saturée de carbonate disodique équivalent à 2,5 fois le poids d'acide oléique. Puis, après décantation et séparation de la phase aqueuse, on lave l'huile à l'eau distillée 2 fois. On la sèche. On la décolore par addition de 6% en poids de terre décolorante pendant 35 mn à 60°C sous azote. On la désodorise pendant 60 mn, à 180°C, à la vapeur, sous un vide de $1.333 \times 10^{-1}$ Pa ($10^{-3}$ Torr).

Dans le tableau I sont présentées les caractéristiques comparatives de deux huiles d'olive, l'une répondant à la définition de la Pharmacopée Européenne, l'autre purifiée selon le procédé de l'invention de l'exemple.

L'huile d'olive obtenue selon le procédé de l'invention est caractérisée par une très faible acidité libre, un très faible indice de peroxyde et une absence quasi totale de pigments (carotenoïdes xanthophylles et chlorophylles).

| | Huile d'olive PH. Europ. 2e Edition II. 10 | Huile d'olive purifiée selon procédé de l'invention |
|---|---|---|
| densité | 0,910 - 0,916 | 0,910 - 0,916 |
| Absorbance U.V. E % 232 nm 270 nm | > 8 | < 3 |
| Indice d'acide | ≤ 2,0 | ≤ 0,2 |
| Indice de peroxyde | ≤ 15,0 | ≤ 1,0 |
| Composition en triglycérides et sterols | identique | |
| Absorbance vis à 382 nm 460 nm 485 nm 660 nm | Absence de norme " " " | ≤ 0,80 ≤ 0,02 ≤ 0,02 ≤ 0,02 |

**Revendications**

1. Procédé de purification de l'huile d'olive, procédé caractérisé en ce que l'on purifie une huile d'olive telle que définie à la Pharmacopée Européenne, c'est-à-dire une huile grasse obtenue à partir des drupes mûres d'Olea europaea L., par pression à froid ou par tout autre moyen mécanique approprié de la manière suivante: on neutralise l'huile de départ par une solution aqueuse saturée de carbonate alcalin, la solution étant en quantité équivalente à deux à dix fois le poids d'acide oléique calculé après dosage, sous atmosphère inerte, à une température comprise entre 20 et 80°C, sous agitation, puis, après décantation et séparation de la phase aqueuse, on lave l'huile jusqu'à neutralité complète, puis, on décolore l'huile après l'avoir séchée à l'aide de terre décolorante, sous atmosphère inerte, à une température comprise entre 20 et 80°C.

2. Procédé selon la revendication 1, caractérisé par le fait que l'on désodorise l'huile obtenue à basse température.

3. Procédé selon la revendication 1 ou la revendication 2, caractérisé par le fait que l'on utilise le carbonate disodique.

4. Procédé selon l'une quelconque des revendications 1 à 3, caractérisé par le fait que l'on utilise une solution aqueuse saturée de carbonate disodique en une quantité équivalente à deux à trois fois le poids d'acide oléique.

5. Procédé selon l'une quelconque des revendications 1 à 4, caractérisé par le fait que l'on utilise une solution saturée de carbonate disodique en une quantité équivalente à deux fois et demie le poids d'acide oléique.

6. Huile d'olive pure obtenue selon le procédé décrit dans l'une quelconque des revendications 1 à 5.

7. Emulsion d'huile d'olive pure obtenue selon le procédé décrit dans l'une quelconque des revendications 1 à 5, destinée à l'alimentation entérale ou parentérale.

8. Emulsion pour l'alimentation entérale ou parentérale, caractérisée par le fait qu'elle contient, parmi ses constituants, de l'huile d'olive purifiée selon le procédé décrit dans l'une quelconque des revendications 1 à 5.

## Patentansprüche

1. Verfahren zur Reinigung von Olivenöl, dadurch gekennzeichnet, daß man ein Olivenöl gemäß der Definition des Europäischen Arzneibuches, d.h. ein fettes Öl, das aus den reifen Früchten von Olea europaea L. durch Kaltpressen oder auf andere geeignete mechanische Weise gewonnen wurde, auf folgende Art reinigt : man neutralisiert das Ausgangsöl mit einer gesättigten wässerigen Alkalicarbonatlösung, deren Menge der zwei- bis zehnfachen Gewichtsmenge der nach der Analyse berechneten Ölsäure äquivalent ist, unter Inertatmosphäre bei einer Temperatur zwischen 20 und 80°C unter Rühren, wäscht dann nach dem Dekantieren und der Abtrennung der wässerigen Phase das Öl bis zur vollständigen Neutralität und entfärbt das Öl, nachdem es getrocknet wurde, mit Bleicherde unter Inertatmosphäre bei einer Temperatur zwischen 20 und 80°C.

2. Verfahren nach Anspruch 1, dadurch gekennzeichnet, daß man das erhaltene Öl bei tiefer Temperatur desodoriert.

3. Verfahren nach Anspruch 1 oder 2, dadurch gekennzeichnet, daß man Dinatriumcarbonat verwendet.

4. Verfahren nach einem der Ansprüche 1 bis 3, dadurch gekennzeichnet, daß man eine gesättigte wässerige Dinatriumcarbonatlösung in einer dem zwei- bis dreifachen Ölsäuregewicht äquivalenten Menge verwendet.

5. Verfahren nach einem der Ansprüche 1 bis 4, dadurch gekennzeichnet, daß man eine gesättigte Dinatriumcarbonatlösung in einer dem zweieinhalbfachen Ölsäuregewicht äquivalenten Menge verwendet.

6. Reines Olivenöl, gewonnen nach dem in einem der Ansprüche 1 bis 5 beschriebenen Verfahren.

7. Emulsion von reinem Olivenöl, das nach dem in einem der Ansprüche 1 bis 5 beschriebenen Verfahren gewonnen wurde, zum Zwecke der enteralen oder parenteralen Ernährung.

8. Emulsion für die enterale oder parenterale Ernährung, dadurch gekennzeichnet, daß sie als Bestandteil Olivenöl enthält, das nach dem in einem der Ansprüche 1 bis 5 beschriebenen Verfahren gereinigt wurde.

## Claims

1. Process for purifying olive oil, which process is characterised in that an olive oil as defined in the European Pharmacopoeia, that is to say a fatty oil obtained from the ripe drupes of Olea europaea L. by cold pressing or by any other suitable mechanical means, is purified in the following manner : the starting oil is neutralised with a saturated aqueous solution of an alkali metal carbonate, the solution being in an amount equivalent to two to ten times the weight of oleic acid calculated after assay, under an inert atmosphere, at a temperature of between 20 and 80°C, with stirring, then, after settling and separation of the aqueous phase, the oil is washed until completely neutral, and the oil is then decolorised after being dried using bleaching earth, under an inert atmosphere, at a temperature of between 20 and 80°C.

2. Process according to Claim 1, characterised in that the oil obtained is deodorised at low temperature.

3. Process according to Claim 1 or Claim 2, characterised in that disodium carbonate is used.

4. Process according to any one of Claims 1 to 3, characterised in that a saturated aqueous solution of disodium carbonate is used in an amount equivalent to two to three times the weight of oleic acid.

5. Process according to any one of Claims 1 to 4, characterised in that a saturated solution of disodium carbonate is used in an amount equivalent to two and a half times the weight of oleic acid.

6. Pure olive oil obtained according to the process described in any one of Claims 1 to 5.

7. Emulsion of pure olive oil obtained according to the process described in any one of Claims 1 to 5, intended for enteral or parenteral feeding.

8. Emulsion for enteral or parenteral feeding, characterised in that it contains, among its constituents, olive oil purified according to the process described in any one of Claims 1 to 5.